# EUROPEAN PATENT APPLICATION

(11) **EP 4 570 194 A1**
(43) Date of publication of application: **18.06.2025**
(21) Application number: 24220181.2
(22) Date of filing: 16.12.2024
(51) Int. Cl.: A61B 17/072, A61B 17/00, A61B 90/00, A61B 17/29

(54) **IMMERSIBLE AND CORROSION RESISTANT SHAFT ASSEMBLIES**

(30) Priority: 15.12.2023 US 202318541712
(71) Applicant: Cilag GmbH International, 6300 Zug (CH)
(72) Inventor: SHELTON IV, Frederick E., Cincinnati, 45242 (US); ADAMS, Shane R., Cincinnati, 45242 (US)
(74) Representative: Carpmaels & Ransford LLP

(57) **Abstract**

The present disclosure relates to slip ring assemblies with passivation layers that increase corrosion resistance for reusable surgical instruments or shaft assemblies for the surgical instruments. Disclosed are shaft assemblies including slip rings with passivation layers formed thereon to prevent corrosion while allowing current flow therethrough.

## Description

### Field of Invention

The present disclosure generally relates to surgical instruments having components with passivation layers. More specifically, the present disclosure relates to slip ring assemblies with passivation layers that increase corrosion resistance for the surgical instruments.

### Background

Surgical instruments are subjected to a number of harsh conditions before, during, and after their intended use. Reusable instruments, for instance, require a sterilization process, which could include subjecting the instrument to high heat and pressure. In addition to or alternatively, chemicals can be added to the surgical instrument to sterilize before use on a patient. During the procedure, the surgical instrument is subjected to a variety of environments, including being exposed to fluids with various amounts of salinity, including blood and saline. After the procedure, a reusable surgical instrument is then sterilized again. During these processes, the surgical instrument can be susceptible to corrosion, which can lead to undesired effects, such as loss of functionality of the surgical instrument. These and other problems exist.

### Summary

It is an object of the present designs to provide devices and methods to meet the above-stated needs. The designs can be for systems and devices for protecting an implantable adjunct on a staple cartridge, while also providing structure to allow compression of the adjunct to the deck of the staple cartridge before being used in surgery.

The instant disclosure describes provides a shaft assembly for a surgical instrument. The shaft assembly comprises a slip ring. The slip ring assembly comprises a first slip ring comprising a first conductor made of a first reactive material, the first reactive material comprising a first passivation layer of oxide formed thereon. The slip ring assembly further comprises a second slip ring comprising a second conductor made of a second reactive material, the second reactive material comprising a second passivation layer of oxide formed thereon. The first passivation layer and the second passivation layer are in direct electrical communication with each other allowing a current to pass from the first conductor to the second conductor.

The instant disclosure describes a method for forming a shaft assembly for a surgical instrument. The method includes forming a first conductor for a first slip ring out of a first reactive material. The method includes forming a first passivation layer of oxide on the first conductor. The method includes forming a second conductor for a second slip ring out of a second reactive material. The method includes forming a second passivation layer of oxide on the second conductor. The method includes assembling the first slip ring and the second slip ring within a surgical instrument such that the first passivation layer and the second passivation layer are in direct electrical communication with each other allowing a current to pass between the first conductor and the second conductor.

Other aspects of the present disclosure will become apparent upon reviewing the following detailed description in conjunction with the accompanying figures. Additional features or manufacturing and use steps can be included as would be appreciated and understood by a person of ordinary skill in the art.

### Brief Description of the Drawings

The above and further aspects of this invention are further discussed with reference to the following description in conjunction with the accompanying drawings, in which like numerals indicate like structural elements and features in various figures. The drawings are not necessarily to scale, emphasis instead being placed upon illustrating principles of the invention. The figures depict one or more implementations of the inventive devices, by way of example only, not by way of limitation. It is expected that those of skill in the art can conceive of and combine elements from multiple figures to better suit the needs of the user.
FIG. 1 is a perspective view of a surgical instrument that has a shaft assembly and an end effector, in accordance with one or more aspects of the present disclosure.
FIG. 2 is an exploded assembly view of a portion of the surgical instrument of FIG. 1, in accordance with one or more aspects of the present disclosure.
FIG. 3 is an exploded view of an end effector of the surgical instrument of FIG. 1, in accordance with one or more aspects of the present disclosure.
FIG. 4 is a perspective view of an RF cartridge and an elongate channel adapted for use with the RF cartridge, in accordance with one or more aspects of the present disclosure.
FIG. 5 is an exploded assembly view of portions of the interchangeable shaft assembly of the surgical instrument of FIG. 1, in accordance with one or more aspects of the present disclosure.
FIG. 6 is another exploded assembly view of portions of the interchangeable shaft assembly of FIG. 1, in accordance with one or more aspects of the present disclosure.
FIG. 7 is a cross-sectional view of a portion of the interchangeable shaft assembly of FIG. 1, in accordance with one or more aspects of the present disclosure.
FIG. 8 is a perspective view of a portion of the shaft assembly of FIG. 1 with the switch drum omitted for clarity, in accordance with one or more aspects of the present disclosure.
FIG. 9 is another perspective view of the portion of the interchangeable shaft assembly of FIG. 1 with the switch drum mounted thereon, in accordance with one or more aspects of the present disclosure.
FIG. 10 is a perspective partial cut-away view of a slip ring assembly, in accordance with one or more aspects of the present disclosure.
FIG. 11 is a cross sectional schematic of a slip ring assembly, in accordance with one or more aspects of the present disclosure.
FIG. 12 is a cross sectional of a slip ring assembly, in accordance with one or more aspects of the present disclosure.
FIG. 13 is a block diagram of a circuit of a surgical instrument, illustrating interfaces between a control circuit, a power source, a slip ring assembly, and an end effector, in accordance with one or more aspects of the present disclosure.
FIG. 14 is a flowchart showing a method of forming a shaft assembly, in accordance with one or more aspects of the present disclosure.

### Detailed Description

Example devices and methods are provided herein for shaft assemblies for surgical instruments that provide a level of corrosion resistance by having an inert passivation layer. Throughout this disclosure, reference will be made to a surgical stapling instrument (e.g., surgical instrument 10) that includes an end effector (e.g., end effector 300) at the end of a shaft assembly (e.g., shaft assembly 200). It will be understood, however, that the solutions provided herein can be incorporated into any other reusable surgical instrument that benefits from a current and/or a signal being passed to and from the distal end of the surgical instrument. Reference to a surgical stapling instrument, therefore, provides an exemplified embodiment, an example that benefits from the solutions provided herein.

Surgical instruments are susceptible to corrosion that can impede their ability to pass signals through the instrument. Most surgical instruments, or any electrical instrument in general, typically have copper on copper (or alloys thereof including zinc, iron, beryllium, etc.) connections. This composition of components can be an issue for surgical instruments, since they are subjected to harsh environments, such as being rinsed or immersed in saline during procedures and then exposed to air to dry. Exposing the instrument to these environments can cause destruction or corrosion of the copper contacts, resulting in signal loss and inconsistent power transmission. Repeated mechanical wear can also exacerbate the problem. Current solutions to these issues revolve around sealing slip rings and contacts in insulative materials, cleaning them with each use or insertion, or coating them with a conductive ink or other coating. But these solutions can also provide disadvantages, since sealing the slip rings and contacts requires fabricating additional components, and conductive inks and coatings can also degrade signal transmission and can also wear down over time. Coating solutions, sealing solutions, non-contact capacitive solutions, wireless connection solutions, and the like all have drawbacks in cost, signal loss, signal sensitivity, complexity, and in general metal damage.

The above issues can improve with the use of metal-on-metal direct contact slip rings. However, when products use direct metal-to-metal contact, they can end up with corrosion of the contacts, destruction of the metals in the presence of ionic fluids (e.g., body fluids acting as an electrolyte for galvanic corrosion), and signal leakage. To illustrate, the electrical connections in most electronic devices can include copper. But said interconnects can also include silver, gold, and platinum. The anodic indexes of copper versus silver, gold, and platinum is such that they are sufficiently far apart to cause galvanic corrosion. Also, silver gold, platinum, and graphite have toxicity and physiologic interactions that make exposed contacts of these materials also less desirable. In general, a solution should include metallic-to-metallic direct contact slip rings, that do not have seals, coatings, or other costly secondary assemblies or interfering coatings to inhibits power transfer and low power data transfer. The preferred solutions would also prevent metal-to-metal destructive interaction.

The present disclosure provides a solution to the above mentioned problems by implementing immersible yet corrosion resistant electrical contact arrangement for medical devices to minimize the impacts on signal and power transmission loss on movable interconnections. The device (e.g., surgical device 10) has a series of slip rings (e.g., first slip ring 2010 and second slip ring 2110) between a handle (e.g., handle assembly 14) and the shaft (e.g., shaft assembly 200) that provide electrical signal and/or power to an end effector (e.g., end effector 300) while maintaining 360° or greater end effector rotation. This is achieved all while the signal and/or power to and from the end effector remains substantially constant. To do so, the present solutions describe components of a surgical instrument that include a passivation layer. This passivation layer can be a natural oxide layer formed on the material of the device components. For example, components made of commercially pure titanium are prone to oxidation when exposed to air (i.e., oxygen). The oxidation creates a layer of titanium oxide on the surface of the material. This oxide layer, which is typically around 1-150nm in thickness, creates a layer of corrosion resistance that impedes further degradation of the material. Other materials, such as tantalum and niobium also react when exposed to air, forming oxide film layers of tantalum pentoxide and niobium oxide, respectively. These tantalum pentoxide and niobium oxide film layers have thicknesses similar to that of the oxide layer of titanium.

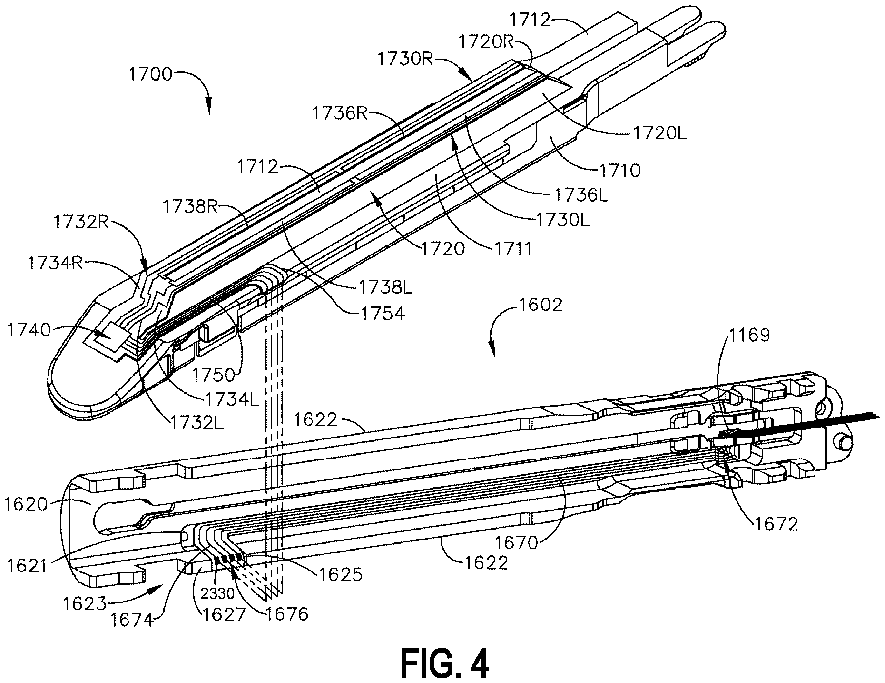

Although the passivation layer can provide corrosion resistance to the component, the thin, generally less than 200nm thick, film can be formed such that the conductivity remains sufficient through the components to pass signals, for example through the shaft assembly to the end effector. As will be described in greater detail with respect to FIG. 4, certain implementations of surgical staplers can include intelligent staple cartridges, for example radio frequency (RF) cartridges, that can send information to the surgical instrument 10 that includes information about the cartridge (e.g., lengths, sizes, and types of staples in the cartridge). Accordingly, the electrical current passing through the device from the end effector to one or more control circuits proximal to the end effector must be sufficient to carry signals even with the passivation layer formed. It has been shown that slip ring assemblies as described herein including commercially pure titanium can generate 3-5 watts at the end effector using a 12V battery, i.e., a current of approximately 0.25A to approximately 0.42A. This ability to carry a current through a slip ring assembly separates the present disclosure from prior designs that use oxide layers as an insulator, or as a capacitive channel-instead of a capacitive channel, the thin-film oxide layer described herein creates corrosion resistance while allowing direct current flow. For instance, certain prior examples described having a capacitive connection to minimize the issue of shorting in the presence of fluid. One issue with such design is the loss of data or a limitation on power or data rate transfers. The present disclosure provides examples by carrying, instead, a current directly through slip rings.

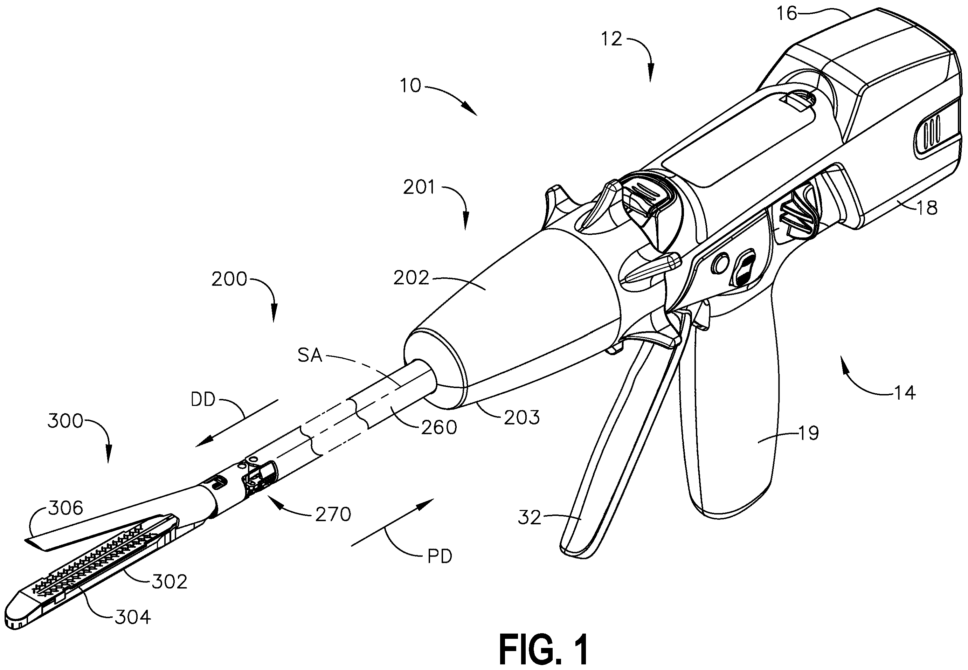

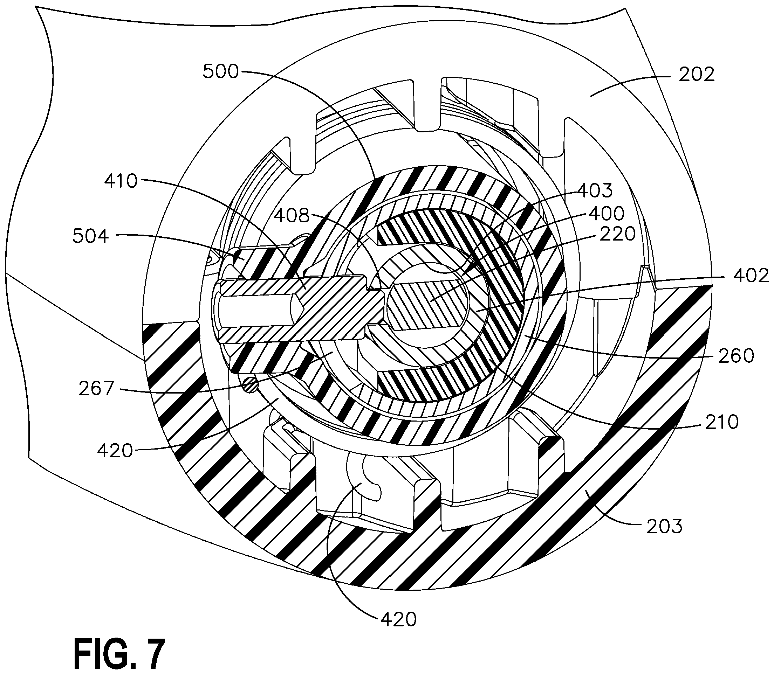

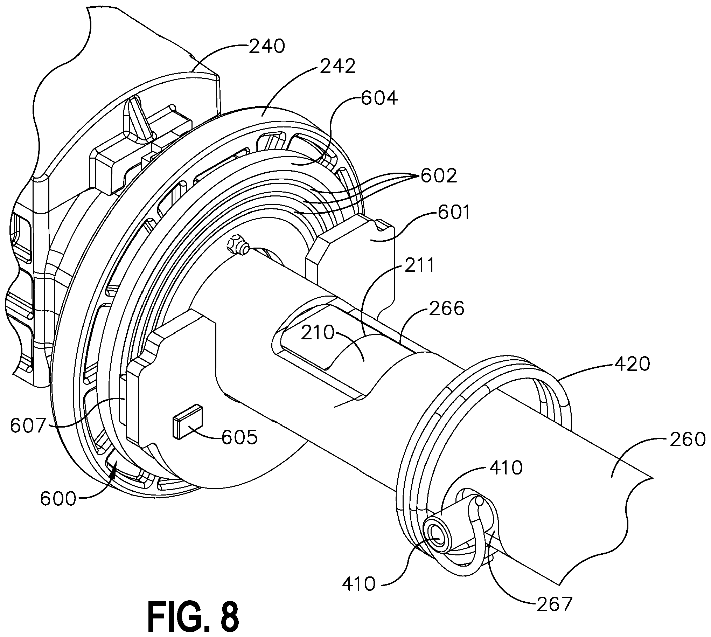

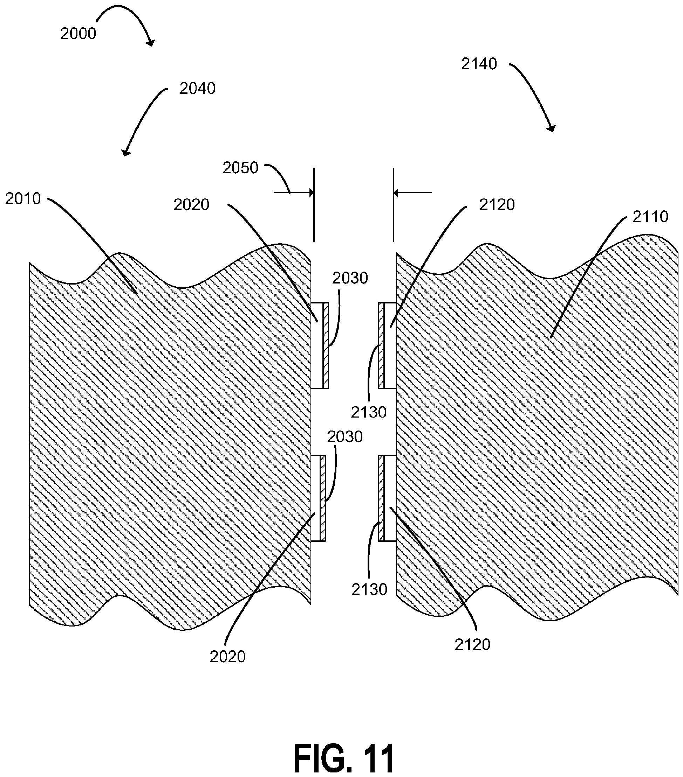

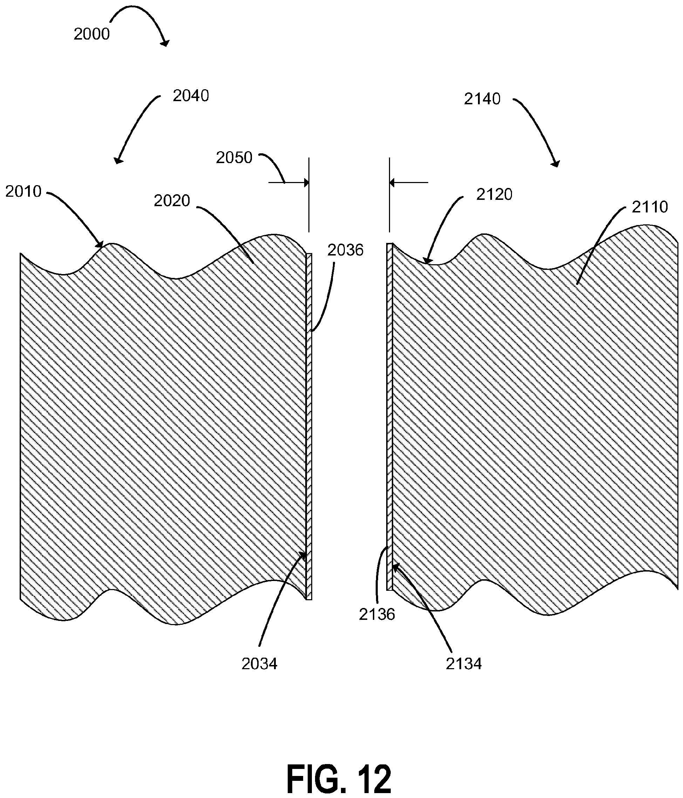

Examples of surgical staplers will now be described for context on how slip ring assemblies can be employed to carry signals to an end effector while also allowing 360°+ rotation of the end effector. FIGs. 1-10 depict a motor-driven surgical instrument 10 for cutting and fastening that can be reused. FIGs. 11 and 12 discuss options and examples to increase the reusability of the surgical instrument 10 with passivation layers. It will also be understood that FIGs. 1-10 provide only illustrative examples of a surgical instrument 10 that can incorporate the anti-corrosion slip ring assembly. Additional information about the embodiments described in FIGs. 1-10 can be found in U.S. Patent Application Publication No. 2019/0000530, which is incorporated herein by reference in its entirety. In the illustrated examples of FIGs. 1-10 in particular, the surgical instrument 10 includes a housing 12 that comprises a handle assembly 14 that is configured to be grasped, manipulated, and actuated by the clinician. The housing 12 is configured for operable attachment to an interchangeable shaft assembly 200 that has an end effector 300 operably coupled thereto that is configured to perform one or more surgical tasks or procedures. In accordance with the present disclosure, various forms of interchangeable shaft assemblies can be effectively employed in connection with robotically controlled surgical systems. The term "housing" may encompass a housing or similar portion of a robotic system that houses or otherwise operably supports at least one drive system configured to generate and apply at least one control motion that could be used to actuate interchangeable shaft assemblies. The term "frame" may refer to a portion of a handheld surgical instrument. The term "frame" also may represent a portion of a robotically controlled surgical instrument and/or a portion of the robotic system that can be used to operably control a surgical instrument.

FIG. 1 is a perspective view of a surgical instrument 10 that has an interchangeable shaft assembly 200 operably coupled thereto according to one aspect of this disclosure. The housing 12 includes an end effector 300 that comprises a surgical cutting and fastening device configured to operably support a surgical staple cartridge 304 therein. The housing 12 can be configured for use in connection with interchangeable shaft assemblies that include end effectors that are adapted to support different sizes and types of staple cartridges, have different shaft lengths, sizes, and types. The housing 12 can be employed with a variety of interchangeable shaft assemblies, including assemblies configured to apply other motions and forms of energy such as, radio frequency (RF) energy, ultrasonic energy, and/or motion to end effector arrangements adapted for use in connection with various surgical applications and procedures. The end effectors, shaft assemblies, handles, surgical instruments, and/or surgical instrument systems can utilize any suitable fastener, or fasteners, to fasten tissue. For instance, a fastener cartridge comprising a plurality of fasteners removably stored therein can be removably inserted into and/or attached to the end effector of a shaft assembly.

The handle assembly 14 can comprise a pair of interconnectable handle housing segments 16, 18 interconnected by screws, snap features, adhesive, etc. The handle housing segments 16, 18 cooperate to form a pistol grip portion 19 that can be gripped and manipulated by the clinician. The handle assembly 14 operably supports a plurality of drive systems configured to generate and apply control motions to corresponding portions of the interchangeable shaft assembly that is operably attached thereto.

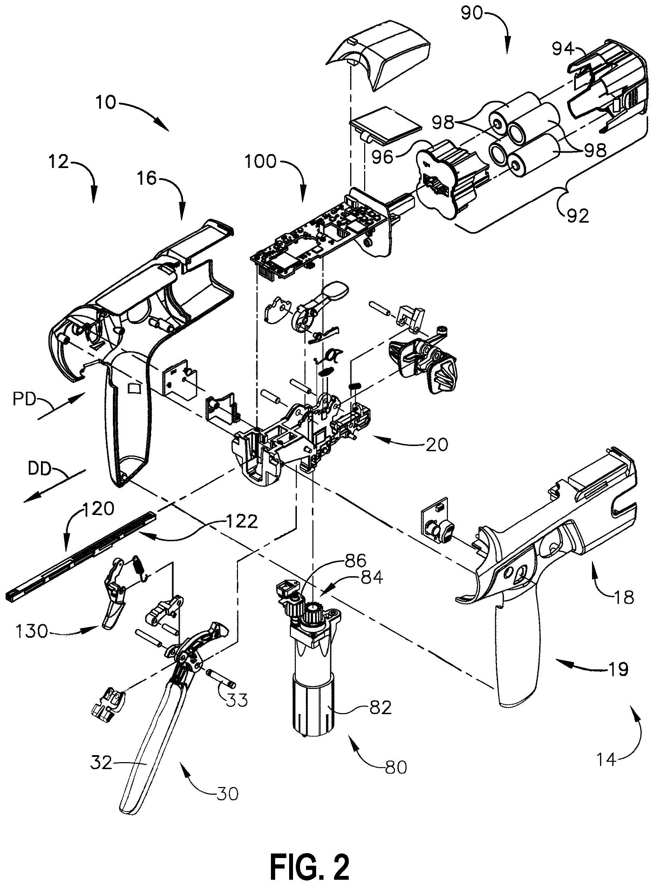

FIG. 2 is an exploded assembly view of a portion of the surgical instrument 10 of FIG. 1 according to one aspect of this disclosure. The handle assembly 14 can include a frame 20 that operably supports a plurality of drive systems. The frame 20 can operably support a "first" or closure drive system 30, which can apply closing and opening motions to the interchangeable shaft assembly 200. The closure drive system 30 can include an actuator such as a closure trigger 32 pivotally supported by the frame 20. The closure trigger 32 is pivotally coupled to the handle assembly 14 by a pivot pin 33 to enable the closure trigger 32 to be manipulated by a clinician. When the clinician grips the pistol grip portion 19 of the handle assembly 14, the closure trigger 32 can pivot from a starting or "unactuated" position to an "actuated" position and more particularly to a fully compressed or fully actuated position.

The handle assembly 14 and the frame 20 can operably support a firing drive system 80 configured to apply firing motions to corresponding portions of the interchangeable shaft assembly attached thereto. The firing drive system 80 can employ an electric motor 82 located in the pistol grip portion 19 of the handle assembly 14. The electric motor 82 can be a DC brushed motor having a maximum rotational speed of approximately 25,000 RPM, for example. In other arrangements, the motor can include a brushless motor, a cordless motor, a synchronous motor, a stepper motor, or any other suitable electric motor. The electric motor 82 can be powered by a power source 90 that can comprise a removable power pack 92. The removable power pack 92 can comprise a proximal housing portion 94 configured to attach to a distal housing portion 96. The proximal housing portion 94 and the distal housing portion 96 are configured to operably support a plurality of batteries 98 therein. Batteries 98 can each comprise, for example, a Lithium Ion (LI) or other suitable battery. The distal housing portion 96 is configured for removable operable attachment to a control circuit board 100, which is operably coupled to the electric motor 82. Several batteries 98 connected in series can power the surgical instrument 10. The power source 90 can be replaceable and/or rechargeable.

The electric motor 82 can include a rotatable shaft (not shown) that operably interfaces with a gear reducer assembly 84 mounted in meshing engagement with a with a set, or rack, of drive teeth 122 on a longitudinally movable drive member 120. The longitudinally movable drive member 120 has a rack of drive teeth 122 formed thereon for meshing engagement with a corresponding drive gear 86 of the gear reducer assembly 84.

In use, a voltage polarity provided by the power source 90 can operate the electric motor 82 in a clockwise direction wherein the voltage polarity applied to the electric motor by the battery can be reversed in order to operate the electric motor 82 in a counterclockwise direction. When the electric motor 82 is rotated in one direction, the longitudinally movable drive member 120 will be axially driven in the distal direction "DD." When the electric motor 82 is driven in the opposite rotary direction, the longitudinally movable drive member 120 will be axially driven in a proximal direction "PD." The handle assembly 14 can include a switch that can be configured to reverse the polarity applied to the electric motor 82 by the power source 90. The handle assembly 14 can include a sensor configured to detect the position of the longitudinally movable drive member 120 and/or the direction in which the longitudinally movable drive member 120 is being moved.

Actuation of the electric motor 82 can be controlled by a firing trigger 130 that is pivotally supported on the handle assembly 14. The firing trigger 130 can be pivoted between an unactuated position and an actuated position.

Turning back to FIG. 1, the interchangeable shaft assembly 200 includes an end effector 300 comprising an elongated channel 302 configured to operably support a surgical staple cartridge 304 therein. The end effector 300 can include an anvil 306 that is pivotally supported relative to the elongated channel 302. The interchangeable shaft assembly 200 can include an articulation joint 270. The interchangeable shaft assembly 200 can include a proximal housing or nozzle 201 comprised of nozzle portions 202, 203. The interchangeable shaft assembly 200 can include a closure tube 260 extending along a shaft axis SA that can be utilized to close and/or open the anvil 306 of the end effector 300.

Turning back to FIG. 1, the closure tube 260 is translated distally (direction "DD") to close the anvil 306, for example, in response to the actuation of the closure trigger 32 in the manner described in U.S. Patent Application Publication No. 2014/0263541, which is incorporated herein by reference in its entirety. The anvil 306 is opened by proximally translating the closure tube 260. In the anvil-open position, the closure tube 260 is moved to its proximal position.

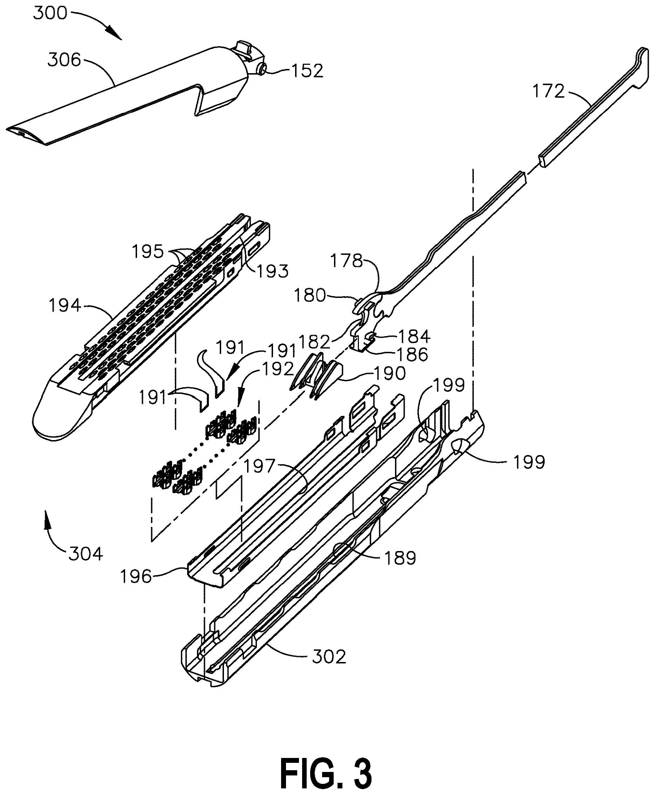

FIG. 3 is an exploded view of one aspect of an end effector 300 of the surgical instrument 10 of FIG. 1 in accordance with one or more aspects of the present disclosure. The end effector 300 can include the anvil 306 and the surgical staple cartridge 304. In this non-limiting example, the anvil 306 is coupled to an elongated channel 302. For example, apertures 199 can be defined in the elongated channel 302 which can receive pins 152 extending from the anvil 306 and allow the anvil 306 to pivot from an open position to a closed position relative to the elongated channel 302 and surgical staple cartridge 304. A firing bar 172 is configured to longitudinally translate into the end effector 300. The firing bar 172 can be constructed from one solid section, or in various examples, can include a laminate material comprising, for example, a stack of steel plates. The firing bar 172 comprises an E-beam 178 and a cutting edge 182 at a distal end thereof. In various aspects, the E-beam can be referred to as an (beam. A distally projecting end of the firing bar 172 can be attached to the E-beam 178 element in any suitable manner and can, among other things, assist in spacing the anvil 306 from a surgical staple cartridge 304 positioned in the elongated channel 302 when the anvil 306 is in a closed position. The E-beam 178 also can include a sharpened cutting edge 182 that can be used to sever tissue as the E-beam 178 is advanced distally by the firing bar 172. In operation, the E-beam 178 also can actuate, or fire, the surgical staple cartridge 304. The surgical staple cartridge 304 can include a molded cartridge body 194 that holds a plurality of staples 191 resting upon staple drivers 192 within respective upwardly open staple cavities 195. A wedge sled 190 is driven distally by the E-beam 178, sliding upon a cartridge tray 196 that holds together the various components of the surgical staple cartridge 304. The wedge sled 190 upwardly cams the staple drivers 192 to force out the staples 191 into deforming contact with the anvil 306 while the cutting edge 182 of the E-beam 178 severs clamped tissue.

The E-beam 178 can include upper pins 180 that engage the anvil 306 during firing. The E-beam 178 can further include middle pins 184 and a bottom foot 186 that can engage various portions of the cartridge body 194, cartridge tray 196, and elongated channel 302. When a surgical staple cartridge 304 is positioned within the elongated channel 302, a slot 193 defined in the cartridge body 194 can be aligned with a longitudinal slot 197 defined in the cartridge tray 196 and a slot 189 defined in the elongated channel 302. In use, the E-beam 178 can slide through the aligned longitudinal slots 193, 197, and 189 wherein, as indicated in FIG. 3, the bottom foot 186 of the E-beam 178 can engage a groove running along the bottom surface of elongated channel 302 along the length of slot 189, the middle pins 184 can engage the top surfaces of cartridge tray 196 along the length of longitudinal slot 197, and the upper pins 180 can engage the anvil 306. In such circumstances, the E-beam 178 can space, or limit the relative movement between, the anvil 306 and the surgical staple cartridge 304 as the firing bar 172 is moved distally to fire the staples from the surgical staple cartridge 304 and/or incise the tissue captured between the anvil 306 and the surgical staple cartridge 304. Thereafter, the firing bar 172 and the E-beam 178 can be retracted proximally allowing the anvil 306 to be opened to release the two stapled and severed tissue portions.

Referring to FIG. 4, in at least one arrangement, an interchangeable shaft assembly can be used in connection with an RF cartridge 1700 as well as a surgical staple/fastener cartridge. The RF cartridge 1700 includes a cartridge body 1710 that is sized and shaped to be removably received and supported in the elongate channel 1602. For example, the cartridge body 1710 can be configured to be removable retained in snap engagement with the elongate channel 1602. In at least one aspect, the cartridge body 1710 includes a centrally disposed elongate slot 1712 that extends longitudinally through the cartridge body to accommodate longitudinal travel of a knife therethrough. The RF cartridge 1700 can store information about the cartridge and relay said information to the surgical instrument 10. The information can include information about the staples therein, including length, size, material, etc. The information can also or alternatively include information about how long the longitudinal row of staples are in the cartridge, so that the surgical instrument 10 can provide an appropriate firing length according to the length of the RF cartridge 1700.

The cartridge body 1710 is formed with a centrally disposed raised electrode pad 1720. The elongate slot 1712 extends through the center of the electrode pad 1720 and serves to divide the pad 1720 into a left pad segment 1720L and a right pad segment 1720R. A right flexible circuit assembly 1730R is attached to the right pad segment 1720R and a left flexible circuit assembly 1730L is attached to the left pad segment 1720L. In at least one arrangement for example, the right flexible circuit 1730R comprises a plurality of wires 1732R that can include, for example, wider wires/conductors for RF purposes and thinner wires for conventional stapling purposes that are supported or attached or embedded into a right insulator sheath/member 1734R that is attached to the right pad 1720R. In addition, the right flexible circuit assembly 1730R includes a "phase one", proximal right electrode 1736R and a "phase two" distal right electrode 1738R. Likewise, the left flexible circuit assembly 1730L comprises a plurality of wires 1732L that can include, for example, wider wires/conductors for RF purposes and thinner wires for conventional stapling purposes that are supported or attached or embedded into a left insulator sheath/member 1734L that is attached to the left pad 1720L. In addition, the left flexible circuit assembly 1730L includes a "phase one", proximal left electrode 1736L and a "phase two" distal left electrode 1738L. The left and right wires 1732L, 1732R are attached to a distal microchip 1740 mounted to the distal end portion of the cartridge body 1710.

The elongate channel 1602 includes a channel circuit 1670 that is supported in a recess 1621 that extends from the proximal end of the elongate channel 1602 to a distal location 1623 in the elongate channel bottom portion 1620. The channel circuit 1670 includes a proximal contact portion 1672 that contacts a distal contact portion 1169 of a flexible shaft circuit strip for electrical contact therewith. A distal end 1674 of the channel circuit 1670 is received within a corresponding wall recess 1625 formed in one of the channel walls 1622 and is folded over and attached to an upper edge 1627 of the channel wall 1622. A series of corresponding exposed contacts 1676 are provided in the distal end 1674 of the channel circuit 1670. An end of a flexible cartridge circuit 1750 is attached to the distal microchip 1740 and is affixed to the distal end portion of the cartridge body 1710. Another end is folded over the edge of the cartridge deck surface 1711 and includes exposed contacts 1754 configured to make electrical contact with the exposed contacts 1676 of the channel circuit 1670. Thus, when the RF cartridge 1700 is installed in the elongate channel 1602, the electrodes as well as the distal micro-chip 1740 are powered and communicate with an onboard circuit board through contact between the flexible cartridge circuit 1750, the flexible channel circuit 1670, a flexible shaft circuit and slip ring assembly.

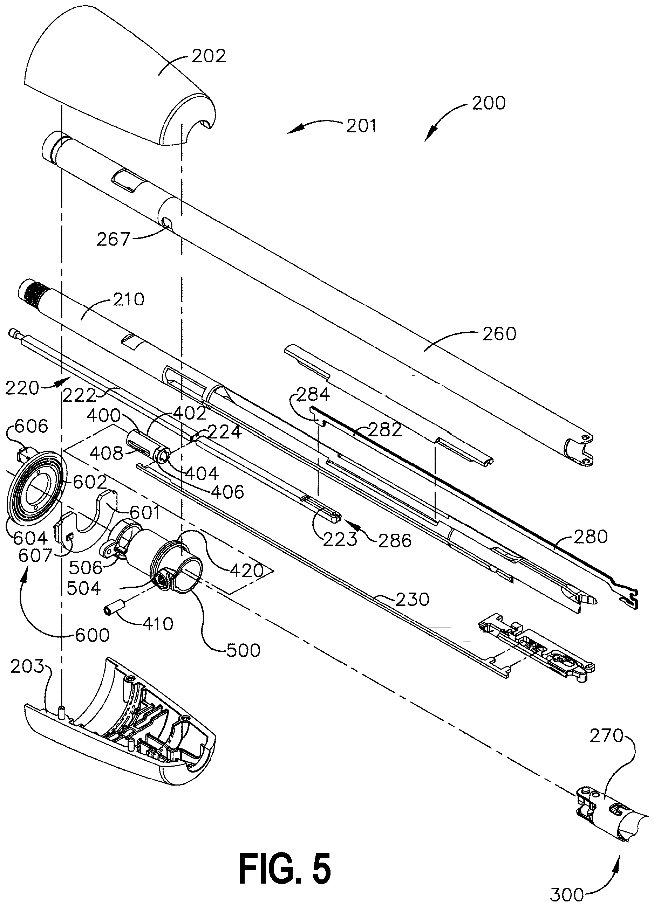

FIG. 5 is another exploded assembly view of portions of the interchangeable shaft assembly 200 according to one aspect of this disclosure. The interchangeable shaft assembly 200 includes a firing member 220 that is supported for axial travel within a shaft spine 210. The firing member 220 includes an intermediate firing shaft portion 222 that is configured for attachment to a distal portion or bar 280. The intermediate firing shaft portion 222 can include a longitudinal slot 223 in the distal end thereof which can be configured to receive a tab 284 on the proximal end 282 of the distal bar 280. The longitudinal slot 223 and the proximal end 282 can be sized and configured to permit relative movement therebetween and can comprise a slip joint 286. The slip joint 286 can permit the intermediate firing shaft portion 222 of the firing member 220 to be moved to articulate the end effector 300 without moving, or at least substantially moving, the bar 280. Once the end effector 300 has been suitably oriented, the intermediate firing shaft portion 222 can be advanced distally until a proximal sidewall of the longitudinal slot 223 comes into contact with the tab 284 in order to advance the distal bar 280. Advancement of the distal bar 280 causes the E-beam 178 to be advanced distally to fire the staple cartridge positioned within the channel 302.

Further to the above, the shaft assembly 200 includes a clutch assembly 400 which can be configured to selectively and releasably couple the articulation drive 230 to the firing member 220. In one form, the clutch assembly 400 includes a lock collar, or sleeve 402, positioned around the firing member 220 wherein the lock sleeve 402 can be rotated between an engaged position in which the lock sleeve 402 couples the articulation drive 230 to the firing member 220 and a disengaged position in which the articulation drive 230 is not operably coupled to the firing member 220. When lock sleeve 402 is in its engaged position, distal movement of the firing member 220 can move the articulation drive 230 distally and, correspondingly, proximal movement of the firing member 220 can move the articulation drive 230 proximally. When lock sleeve 402 is in its disengaged position, movement of the firing member 220 is not transmitted to the articulation drive 230 and, as a result, the firing member 220 can move independently of the articulation drive 230.

The lock sleeve 402 can comprise a cylindrical, or an at least substantially cylindrical, body including a longitudinal aperture 403 defined therein configured to receive the firing member 220. The lock sleeve 402 can comprise diametrically-opposed, inwardly-facing lock protrusions 404 and an outwardly-facing lock member 406. The lock protrusions 404 can be configured to be selectively engaged with the firing member 220. More particularly, when the lock sleeve 402 is in its engaged position, the lock protrusions 404 are positioned within a drive notch 224 defined in the firing member 220 such that a distal pushing force and/or a proximal pulling force can be transmitted from the firing member 220 to the lock sleeve 402. When the lock sleeve 402 is in its engaged position, the second lock member 406 is received within a drive notch 232 defined in the articulation drive 230 such that the distal pushing force and/or the proximal pulling force applied to the lock sleeve 402 can be transmitted to the articulation drive 230. In effect, the firing member 220, the lock sleeve 402, and the articulation drive 230 will move together when the lock sleeve 402 is in its engaged position. On the other hand, when the lock sleeve 402 is in its disengaged position, the lock protrusions 404 may not be positioned within the drive notch 224 of the firing member 220 and, as a result, a distal pushing force and/or a proximal pulling force may not be transmitted from the firing member 220 to the lock sleeve 402. Correspondingly, the distal pushing force and/or the proximal pulling force may not be transmitted to the articulation drive 230. In such circumstances, the firing member 220 can be slid proximally and/or distally relative to the lock sleeve 402 and the proximal articulation drive 230.

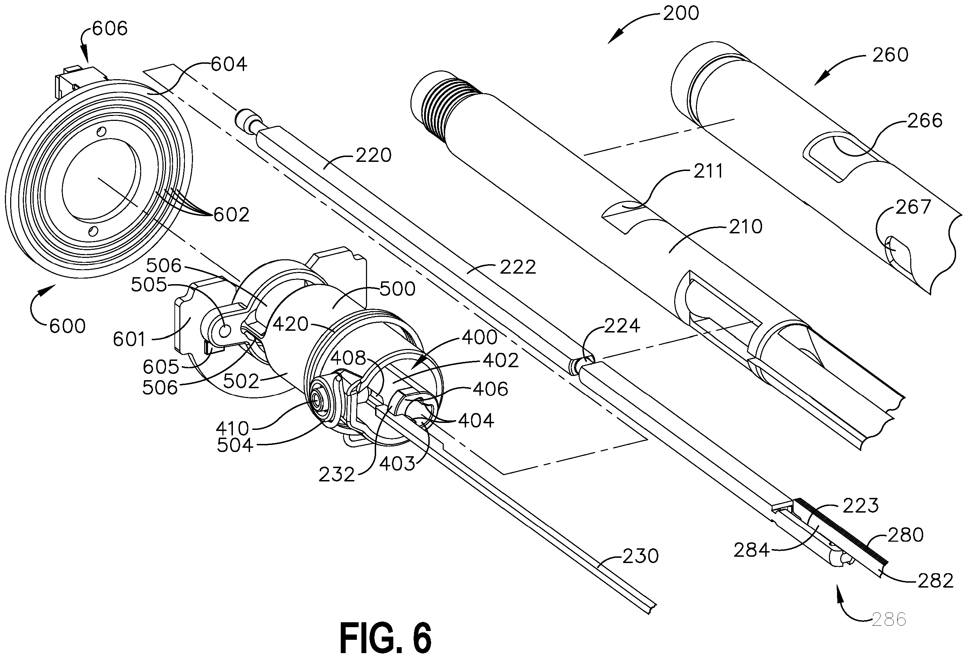

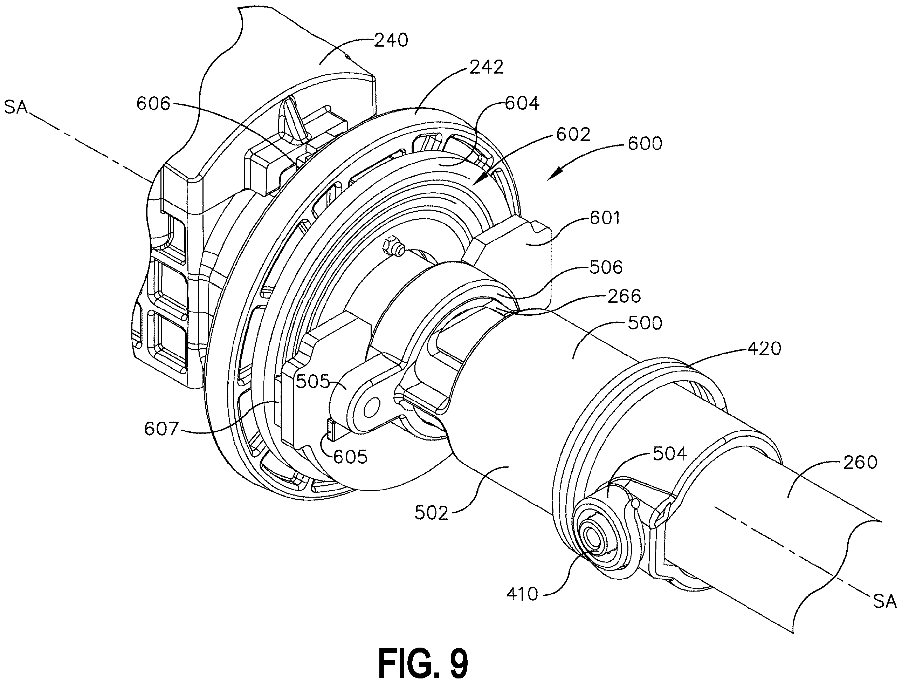

The shaft assembly 200 further includes a switch drum 500 that is rotatably received on the closure tube 260. The switch drum 500 comprises a hollow shaft segment 502 that has a shaft boss 504 formed thereon for receive an outwardly protruding actuation pin 410 therein. In various circumstances, the actuation pin 410 extends through a slot 267 into a longitudinal slot 408 provided in the lock sleeve 402 to facilitate axial movement of the lock sleeve 402 when it is engaged with the articulation drive 230. A rotary torsion spring 420 is configured to engage the boss 504 on the switch drum 500 and a portion of the nozzle housing 203 as shown in FIG. 5 to apply a biasing force to the switch drum 500. The switch drum 500 can further comprise at least partially circumferential openings 506 defined therein which, referring to FIGs. 5 and 6, can be configured to receive circumferential mounts extending from the nozzle halves 202, 203 and permit relative rotation, but not translation, between the switch drum 500 and the proximal nozzle 201. The mounts also extend through openings 266 in the closure tube 260 to be seated in recesses 211 in the shaft spine 210. However, rotation of the nozzle 201 to a point where the mounts reach the end of their respective openings 506 in the switch drum 500 will result in rotation of the switch drum 500 about the shaft axis SA-SA (see FIG. 9). Rotation of the switch drum 500 will ultimately result in the rotation of the actuation pin 410 and the lock sleeve 402 between its engaged and disengaged positions.

The shaft assembly 200 can comprise a slip ring assembly 600 which can be configured to conduct electrical power to and/or from the end effector 300 and/or communicate signals to and/or from the end effector 300, for example. The slip ring assembly 600 can comprise a proximal connector flange 604 mounted to a chassis flange 242 extending from the chassis 240 and a distal connector flange 601 positioned within a slot defined in the nozzle halves 202, 203. The proximal connector flange 604 can comprise a first face and the distal connector flange 601 can comprise a second face which is positioned adjacent to and movable relative to the first face. The distal connector flange 601 can rotate relative to the proximal connector flange 604 about the shaft axis SA-SA. The proximal connector flange 604 can comprise a plurality of concentric, or at least substantially concentric, conductors 602 defined in the first face thereof. A connector 607 can be mounted on the proximal side of the connector flange 601 and can have a plurality of contacts, wherein each contact corresponds to and is in electrical contact with one of the conductors 602. Such an arrangement permits relative rotation between the proximal connector flange 604 and the distal connector flange 601 while maintaining electrical contact therebetween. The proximal connector flange 604 can include an electrical connector 606 which can place the conductors 602 in signal communication with a circuit board mounted to the shaft chassis 240, for example.

The shaft assembly 200 can include a proximal portion which is fixably mounted to the handle assembly 14 and a distal portion which is rotatable about a longitudinal axis. The rotatable distal shaft portion can be rotated relative to the proximal portion about the slip ring assembly 600. The distal connector flange 601 of the slip ring assembly 600 can be positioned within the rotatable distal shaft portion. Moreover, further to the above, the switch drum 500 can also be positioned within the rotatable distal shaft portion. When the rotatable distal shaft portion is rotated, the distal connector flange 601 and the switch drum 500 can be rotated synchronously with one another. In addition, the switch drum 500 can be rotated between a first position and a second position relative to the distal connector flange 601. When the switch drum 500 is in its first position, the articulation drive system can be operably disengaged from the firing drive system and, thus, the operation of the firing drive system may not articulate the end effector 300 of the shaft assembly 200. When the switch drum 500 is in its second position, the articulation drive system can be operably engaged with the firing drive system and, thus, the operation of the firing drive system can articulate the end effector 300 of the shaft assembly 200. When the switch drum 500 is moved between its first position and its second position, the switch drum 500 is moved relative to distal connector flange 601.

In various examples, the shaft assembly 200 can comprise at least one sensor configured to detect the position of the switch drum 500. The distal connector flange 601 can comprise a Hall effect sensor 605, for example, and the switch drum 500 can comprise a magnetic element, such as permanent magnet 505, for example. The Hall effect sensor 605 can be configured to detect the position of the permanent magnet 505. When the switch drum 500 is rotated between its first position and its second position, the permanent magnet 505 can move relative to the Hall effect sensor 605. In various examples, Hall effect sensor 605 can detect changes in a magnetic field created when the permanent magnet 505 is moved. The Hall effect sensor 605 can be in signal communication with a control circuit, for example. Based on the signal from the Hall effect sensor 605, a microcontroller on the control circuit can determine whether the articulation drive system is engaged with or disengaged from the firing drive system.

A surgical instrument may not be able to use a rotatable shaft assembly effectively by using general wires to communicate power and signals between a fixed shaft portion and a rotatable shaft portion of the shaft assembly because the wires may get twisted or even damaged due to the repeated rotation of the shaft assembly. One way to overcome this deficiency can be to use a ring assembly instead of wires to communicate power and signals to the rotatable shaft portion. For example, a first flange with electrodes can be attached to the fixed shaft portion and a second flange with electrodes can rotate relative to the electrodes of the first flange. A gap is necessarily formed between the first flange and the second flange to permit the rotation of the second flange relative to the first flange. In order to maintain an electrical connection during the rotation of the rotatable shaft portion, the electrodes of the first and second flanges can be exposed at an interface therebetween. The gap can permit water and/or other body fluid ingress into the area between the first and second flanges where the electrode interface resides. Accordingly, the electrode interface may become exposed to water and other body fluids during surgery. Upon touching the exposed electrodes, the water and/or body fluids may cause signal noise or even loss of power/signals.

Aspects of the present disclosure improve slip ring assemblies in surgical instruments that that are exposed to water and/or body fluids during their operation. In one arrangement, a shaft assembly can include a proximal shaft portion that can be fixably connected to a body of a surgical instrument and a distal shaft portion rotatable relative to the proximal shaft portion. The slip ring assembly can include a proximal slip ring in the proximal shaft portion and a distal slip ring in the shaft distal portion. Each of the proximal slip ring and the distal slip ring can include one or more conductors mounted on each of the proximal and distal slip rings.

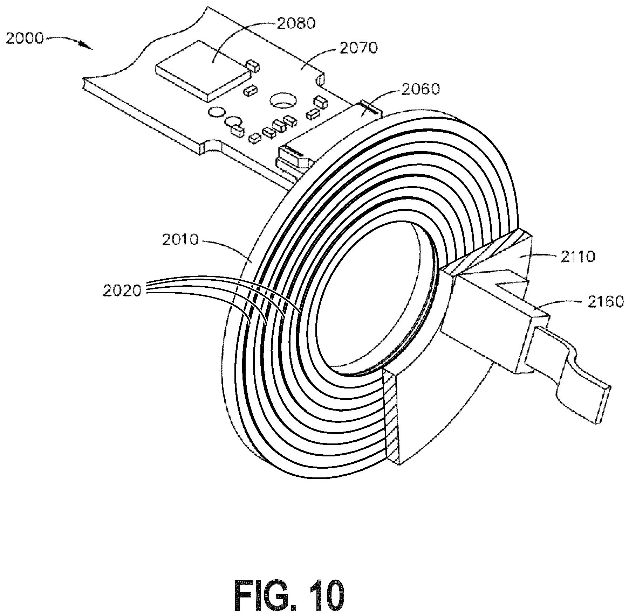

FIG. 10 shows a perspective partial cut-away view of a slip ring assembly 2000 according to one aspect of this disclosure. The slip ring assembly 2000 can be substantially similar to slip ring assembly 600 described above. The slip ring assembly 2000 can be configured to conduct electrical power to and/or from an end effector (e.g., end effector 300) and/or communicate signals to and/or from the end effector. The slip ring assembly can include a proximal slip ring 2010 and a distal slip ring 2110. The proximal slip ring 2010 includes one or more conductors 2020 mounted on the proximal slip ring 2010. The distal slip ring 2110 includes one or more conductors 2120 mounted on the distal slip ring 2110, conductors 2020 being able to pass signals to conductors 2120. The proximal slip ring 2010 can be fixably connected to a body (e.g., handle assembly 14 or a chassis flange 242 of a proximal shaft portion of a shaft assembly) of a surgical instrument (e.g., surgical instrument 10). The distal slip ring 2110 can be fixedly connected to a distal shaft portion of a shaft assembly. The distal slip ring 2110 can be rotatable relative to the proximal slip ring 2010, for example, about a longitudinal axis.

The proximal and distal slip rings 2010, 2110 can be positioned within a slot defined in nozzle halves (e.g., nozzle halves 202, 203, see FIG. 5). The distal slip ring 2110 may rotate relative to the proximal slip ring 2010 about the shaft axis SA-SA. In an example aspect, the thickness of the conductors 2020 (or conductors 2120) can be in the range of about 0.001 inches to about 0.01 inches, preferably in the range of about 0.003 inches to about 0.008 inches, more preferably in the range of about 0.004 inches to about 0.006 inches. In another example aspect, the conductors 2020, 2120 may have any other suitable thickness.

The proximal slip ring 2010 can be fixably connected to the body of the surgical instrument. For example, the proximal slip ring 2010 and the conductors 2020 of the proximal slip ring 2010 can be connected to a shaft circuit board 2070 through a first electrical connector 2060 (e.g., electrical connector 606) as illustrated in FIG. 10. The circuit board 2070 can include a control circuit 2080 (e.g., a microchip or a microprocessor) configured to control the power and signals delivered to an end effector (e.g., end effector 300). The distal slip ring 2110 and the conductors 2120 of the distal slip ring 2110 can be connected to the end effector through a second electrical connector 2160. The control circuit 2080 can be configured to communicate the power and signals (e.g., data or any other signals).

In an example aspect, the first and second slip rings 2010, 2110 can be in a ring shape as illustrated in FIG. 10. In another example aspect, the first and second slip rings 2010, 2110 can have any other suitable shape. In an example aspect, the conductors 2020, 2120 can comprise a metallic electrode. In another example aspect, the conductors 2020, 2120 can comprise any other electrically conductive material. In an example aspect, each of the conductors 2020 on the proximal slip ring 2010 can be matched with one of the conductors 2120 on the distal slip ring 2110 and the matched conductors can be facing each other.

FIG. 11 is a cross sectional schematic of a slip ring assembly 2000 with conductors having passivation layers of oxide, in accordance with one or more aspects of the present disclosure. Referring first to the proximal portion 2040 of the slip ring assembly 200, the assembly can include a first slip ring 2010. The first slip ring 2010 can include a conductor 2020 as described above. In this implementation, the conductor 2020 is made from a reactive material. A "reactive" material as used herein is a material that forms an oxide layer when subjected to air (i.e., oxygen), as shown in the figure as passivation layer 2030. The reactive material can be one of titanium, tantalum, and niobium, and the passivation layer 2030 is the respective oxide film of at least one of titanium oxide, tantalum pentoxide, and niobium oxide. The reactive material is not limited to titanium, tantalum, or niobium, however. As will be appreciated, other materials can create a natural passivation later when exposed to oxygen and/or moisture. As described above, the oxide layer of the materials can be in the order of less than 200nm, e.g., less than 150nm, less than 100nm, less than 50nm, etc. As such, the passivation layer 2030 can create an anti-corrosive barrier to the conductor 2020, while also allowing a current to pass through the conductor 2020. Referring now to the distal portion 2140 of the slip ring assembly 200, the assembly can include a second slip ring 2110. The second slip ring 2110 can include a conductor 2120 as described above. In this implementation, the conductor 2120 is also made from a reactive material that forms an oxide layer when exposed to air (i.e., oxygen), as shown in the figure as passivation layer 2130. The reactive material can be one of titanium, tantalum, and niobium, and the passivation layer 2130 is the respective oxide film of at least one of titanium oxide, tantalum pentoxide, and niobium oxide. As described above, the oxide layer of the materials can be in the order of less than 200nm, e.g., less than 150nm, less than 100nm, less than 50nm, etc. As such, the passivation layer 2030 can create an anti-corrosive barrier to the conductor 2120, while also allowing a current to pass through the conductor 2120.

In some examples, the first conductor 2020 and second conductor 2120 can comprise the same reactive material so as to shield the junction between the first slip ring 2010 (i.e., the proximal slip ring) and second slip ring 2110 (i.e., the distal slip ring) from galvanic corrosion. For example, the surgical instrument 10 will be exposed to conditions with high salinity (e.g., saline to rinse the instrument after use), and this environment can provide the electrolyte necessary to cause the galvanic corrosion of the anode material. The risk for galvanic corrosion can be reduced if both reactive materials are the same. In any of the implementations described herein, the first reactive material and the second reactive material of the first slip ring 2010 and the second slip ring 2120 can have a surface polish to increase passivation. For example, a surface polish can remove contaminants such as iron or other elements from the surface of the component, thereby allowing the oxidation layer to form consistently and uniformly across the surface of the reactive materials. The surface smoothness can include for example a smoothness of 4 Ra micro-inches or better.

In any of the embodiments described herein, including those described in FIGs. 11 and 12, the passivation layers on the reactive materials can be formed during use of the surgical instrument 10, and in some instances can become thicker during use of the surgical instrument. As stated above, surgical instruments such as staplers are subjected to environments that can be harsh on metallic components, including being exposed to blood and saline during procedures. As the surgical instrument 10 is used, the passivation layers of the reactive materials described herein can be formed and/or grow thicker. Further, it has been shown that slip ring assemblies that include the passivation layers described herein can pass signals and/or power to and from the end effector (e.g., end effector 300) that remains substantially constant as a thickness of the passivation layers (e.g., first passivation layer 2030 and second passivation layer 2130) increases. Additionally or alternatively to forming the passivation layers during use, the passivation layers (e.g., first passivation layer 2030 and second passivation layer 2130) can be pre-formed. Pre-forming the passivation layers means allowing the oxide film to form prior to packaging the surgical instrument 10 and sending the final product to the end user.

The example slip ring assembly 2000 shown in FIG. 11 includes conductors 2020, 2120 that are full rings that follow the circumference of the respective slip ring 2010, 2110. These rings sit upon the ends of the respective slip rings 2010, 2110. The first passivation layer 2030 and the second passivation layer 2130 are in direct electrical communication with each other. That is to say, a gap 2050 may exist between the two passivation layers, meaning that air and fluids (e.g., saline, blood, etc.) may be positioned and/or pass between the two passivation layers 2030, 2130. However, the design is such that no solid components are manufactured to be placed between the two passivation layers 2030, 2130 (for example no solid coating, water resistant membrane, insulator, etc. is placed between the two passivation layers).

Referring now to FIG. 12, the example shown therein provides an alternative to the ring-style embodiment shown in FIG. 11 wherein the conductors are separate components positioned on the surface of the respective slip rings. The example shown in FIG. 12 instead comprises a first slip ring 2010 wherein the first conductor 2020 is the entire body of the first slip ring 2010. In this example, the entire first slip ring 2010 can be manufactured from the selected reactive material, meaning that a first passivation layer 2036 will form on the entirety of the slip ring. The distal end 2034 of the first slip ring 2010 can also include the passivation layer 2036. The slip ring assembly 2000 can also include a second slip ring 2110 wherein the second conductor 2120 is the entire body of the second slip ring 2110. In this example, the entire second slip ring 2110 can be manufactured from the selected reactive material, meaning that a second passivation layer 2136 will form on the entirety of the slip ring. The proximal end 2134 of the second slip ring 2110 can also include the passivation layer 2136. Accordingly, the current can pass from the first passivation layer 2036 at the distal end 2034 of the first slip ring 2010 to the second passivation layer 2136 at the proximal end 2134 of the second slip ring 2110.

As described above, the present disclosure is not limited to only slip ring assemblies 2000 comprising reactive materials with passivation layers. Other components, particularly those that are most likely to be subjected to blood, tissue, and saline, can also be manufactured from one or more reactive materials so as to increase corrosion resistance and allow the surgical instrument 10 to be used more than one time. For instance, and referring to FIG. 4, the elongate channel 1602 of the end effector 300 can include a channel circuit 1670 that relays signals and/or power between the distal end of the end effector 300 to one or more control circuits proximal to the end effector 300. As stated above, a series of corresponding exposed contacts 1676 are provided in the distal end 1674 of the channel circuit 1670. These exposed contacts 1676 can be in electrical communication with exposed contacts 1754 on an RF cartridge 1700 such that information from the RF cartridge 1700 can be related to the corresponding control circuits (information such as lengths, sizes, and types of staples in the cartridge). Because these exposed contacts 1676 on the end effector 300 can be subjected to the high-salinity environment, the contacts can also include a reactive material. The reactive material for the exposed contacts 1676 can include a third passivation layer 2330 of oxide formed thereon.

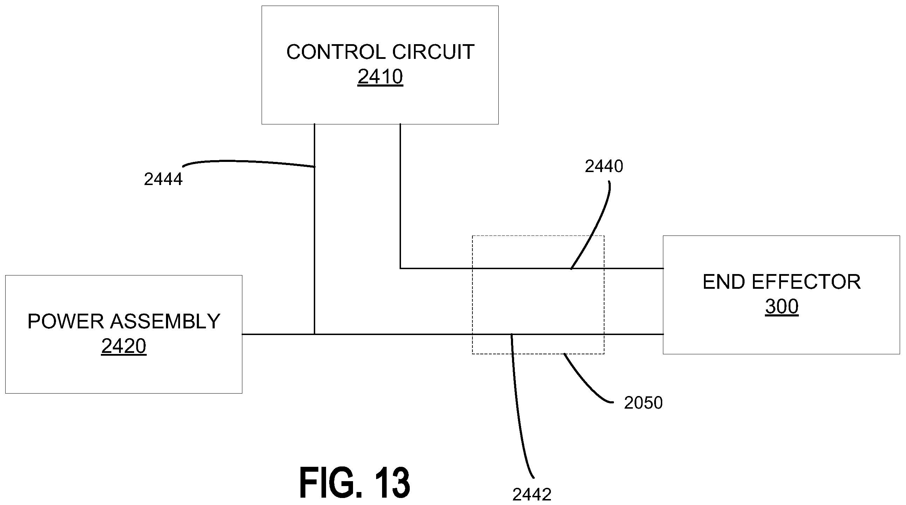

FIG. 13 is a block diagram of a circuit of a surgical instrument 10, illustrating interfaces between a control circuit 2410, a power assembly 2420 (e.g., power source 90), a slip ring assembly 2000, and an end effector 300, in accordance with one or more aspects of the present disclosure. As illustrated in FIG. 13, the slip ring assembly 2000 may be in direct electrical communication with the control circuit 2410 via a first electrical connection 2440. The power assembly 2420 can be in direct electrical communication with the end effector 300 via a second electrical connection 2442, and the power assembly 2420 can be in direct electrical communication with the control circuit 2410 via a third electrical connection 2444. As described above, the electrical communication between the end effector 300 and the control circuit 2410 and power assembly 2420 can be made through the gap 2050 in the slip ring assembly 2000 (see FIGs. 11 and 12).

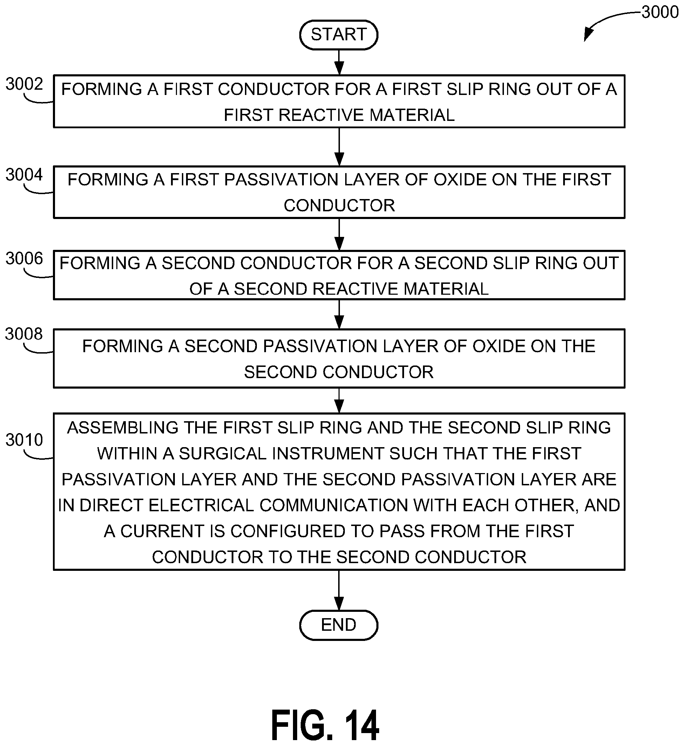

FIG. 14 is a flowchart showing a method 3000 of forming a shaft assembly, in accordance with one or more aspects of the present disclosure. Method 3000 can start with firming 3002 first conductor (e.g., first conductor 2020) for a first slip ring (e.g., first slip ring 2010) out of a first reactive material. Method 3000 includes forming 3004 a first passivation layer (e.g., first passivation layer 2030 or first passivation layer 2036) of oxide on the first conductor. As stated above, this can step can be completed by subjecting the components of the surgical instrument 10 to oxygen rich environments before sending the surgical instrument 10 to the end user; however, the oxide film (e.g., passivation layer) can also be created and/or thicken during use of the product, for example during a surgery and during sterilization of the shaft assembly.

Method 3000 includes forming 3006 a second conductor (e.g., second conductor 2120) for a second slip ring (e.g., second slip ring 2110) out of a second reactive material. Method 3000 includes forming 3008 a second passivation layer (e.g., second passivation layer 2130 or second passivation layer 2136) of oxide on the second conductor. Method 3000 includes assembling 3010 the first slip ring and the second slip ring within a surgical instrument 10 such that the first passivation layer and the second passivation layer are in direct electrical communication with each other allowing a current to pass between the first conductor and the second conductor. Method 3000 can end after step 3010, or other steps can be performed according to the examples described herein.

Examples of the present disclosure can be implemented by any of the following numbered clauses:
Clause 1. A shaft assembly (200) comprising: a slip ring assembly (2000) comprising: a first slip ring (2010) comprising a first conductor (2020) made of a first reactive material, the first reactive material comprising a first passivation layer (2030, 2036) of oxide formed thereon; and a second slip ring (2110) comprising a second conductor (2120) made of a second reactive material, the second reactive material comprising a second passivation layer (2130, 2136) of oxide formed thereon, wherein the first passivation layer (2030, 2036) and the second passivation layer (2130, 2136) are in direct electrical communication with each other allowing a current to pass from the first conductor (2020) to the second conductor (2120).
Clause 2. The shaft assembly (200) of Clause 1, wherein the first reactive material and the second reactive material are the same material.
Clause 3. The shaft assembly (200) of Clause 1 or 2, wherein the first reactive material is selected from the group consisting of titanium, tantalum, and niobium, and wherein the first passivation layer (2030, 2036) is a respective oxide film of at least one of titanium oxide, tantalum pentoxide, and niobium oxide.
Clause 4. The shaft assembly (200) of any one of the preceding Clauses, wherein the first passivation layer (2030, 2036) and the second passivation layer (2130, 2136) are each less than 200nm in thickness.
Clause 5. The shaft assembly (200) of any one of the preceding Clauses, wherein the first conductor (2020) is a first conductive ring around a circumference of the first slip ring (2010) and the second conductor (2120) is a second conductive ring around a circumference of the second slip ring (2110).
Clause 6. The shaft assembly (200) of any one of Clauses 1 to 7, wherein the first conductor (2020) is a first surface of a distal end (2034) of the first slip ring (2010), and the second conductor (2120) is a second surface of a proximal end (2134) of the second slip ring (2110).
Clause 7. The shaft assembly (200) of any one of the preceding Clauses, wherein the first reactive material and the second reactive material have a surface polish to increase passivation.
Clause 8. The shaft assembly (200) of any one of the preceding Clauses, wherein the shaft assembly (200) is disposed within a multi-use surgical instrument (10), and wherein the first conductor (2020) and the second conductor (2120) are exposed to an external environment allowing the first passivation layer (2030, 2036) and the second passivation layer (2130, 2136) to form and/or increase in thickness.
Clause 9. The shaft assembly (200) of Clause 8, wherein the external environment comprises saline, and the first passivation layer (2030, 2036) and the second passivation layer (2130, 2136) are configured to form and/or increase in thickness when subjected to the saline.
Clause 10. The shaft assembly (200) of any one of the preceding Clauses further comprising: a control circuit (2080) electrically connected to the first conductor (2020); and an end effector (300) electrically connected to the second conductor (2120), wherein the control circuit (2080) is configured to communicate signals and/or power to and from the end effector (300) via the current passing between the first conductor (2020) and the second conductor (2120).
Clause 11. The shaft assembly (200) of Clause 10, wherein the signals and/or power remain substantially constant as a thickness of the first passivation layer (2030, 2036) and the second passivation layer (2130, 2136) increases.
Clause 12. The shaft assembly (200) of any one of the preceding Clauses, wherein the current is from approximately 0.25A to approximately 0.42A.
Clause 13. The shaft assembly (200) of any one of the preceding Clauses, wherein the first passivation layer (2030, 2036) and the second passivation layer (2130, 2136) are pre-formed.
Clause 14. The shaft assembly (200) of any one of Clauses 10 to 13, wherein the end effector (300) comprises a channel circuit (1670) and exposed contacts (1676) disposed thereon, wherein the exposed contacts (1676) comprise a third reactive material, third reactive material comprising a third passivation layer (2330) of oxide formed thereon.
Clause 15. The shaft assembly (200) of Clause 14, wherein the first reactive material and the third reactive material are the same material.
Clause 16. A method of forming a shaft assembly (200) comprising: forming a first conductor (2020) for a first slip ring (2010) out of a first reactive material; forming a first passivation layer (2030, 2036) of oxide on the first conductor (2020); forming a second conductor (2120) for a second slip ring (2110) out of a second reactive material; forming a second passivation layer (2130, 2136) of oxide on the second conductor (2120); and assembling the first slip ring (2010) and the second slip ring (2110) within a surgical instrument (10) such that the first passivation layer (2030, 2036) and the second passivation layer (2130, 2136) are in direct electrical communication with each other allowing a current to pass between the first conductor (2020) and the second conductor (2120).
Clause 17. The method of Clause 16 further comprising: electrically connecting the first conductor (2020) to a control circuit (2080); and electrically connecting the second conductor (2120) to an end effector (300), wherein the control circuit (2080) is configured to communicate signals and/or power to and from the end effector (300) via the current passing between the first conductor (2020) and the second conductor (2120).
Clause 18. The method of Clause 16 or 17, wherein the step of electrically connecting the second conductor (2120) to an end effector (300) comprises electrically connecting the second conductor (2120) to a channel circuit (1670) within an elongate channel (1602) of the end effector (300).
Clause 19. The method of Clause 18, wherein the channel circuit (1670) comprises exposed contacts (1676) disposed thereon, wherein the exposed contacts (1676) comprise a third reactive material, and the method further comprises forming a third passivation layer (2330) of oxide on the exposed contacts (1676).
Clause 20. The method of any one of Clauses 16 to 19, wherein the first reactive material is selected from the group consisting of titanium, tantalum, and niobium, and wherein the first passivation layer (2030, 2036) is a respective oxide film of at least one of titanium oxide, tantalum pentoxide, and niobium oxide.

The invention is not necessarily limited to the examples described, which can be varied in construction and detail. The terms "distal" and "proximal" are used throughout the preceding description and are meant to refer to positions and directions relative to the handle of surgical instrument 10. As such, "distal" or distally" refer to a position distant to or a direction away from the handle of surgical instrument 10 (i.e., a direction toward a patient). Similarly, "proximal" or "proximally" refer to a position near or a direction towards the handle of surgical instrument 10 (i.e., toward an operator of the handle). Furthermore, the singular forms "a," "an," and "the" include plural referents unless the context clearly dictates otherwise. Furthermore, the use of "couple", "coupled", or similar phrases should not be construed as being limited to a certain number of components or a particular order of components unless the context clearly dictates otherwise.

As used herein, the terms "about" or "approximately" for any numerical values or ranges indicate a suitable dimensional tolerance that allows the part or collection of components to function for its intended purpose as described herein. More specifically, "about" or "approximately" may refer to the range of values ±10% of the recited value, e.g., "about 90%" may refer to the range of values from 80.1% to 99.9%.

In describing example embodiments, terminology has been resorted to for the sake of clarity. As a result, not all possible combinations have been listed, and such variants are often apparent to those of skill in the art and are intended to be within the scope of the claims which follow. It is intended that each term contemplates its broadest meaning as understood by those skilled in the art and includes all technical equivalents that operate in a similar manner to accomplish a similar purpose without departing from the scope and spirit of the invention. It is also to be understood that the mention of one or more steps of a method does not preclude the presence of additional method steps or intervening method steps between those steps expressly identified. Similarly, some steps of a method can be performed in a different order than those described herein without departing from the scope of the disclosed technology.

## Claims

1. A shaft assembly (200) comprising:
a slip ring assembly (2000) comprising:
a first slip ring (2010) comprising a first conductor (2020) made of a first reactive material, the first reactive material comprising a first passivation layer (2030, 2036) of oxide formed thereon; and
a second slip ring (2110) comprising a second conductor (2120) made of a second reactive material, the second reactive material comprising a second passivation layer (2130, 2136) of oxide formed thereon,
wherein the first passivation layer (2030, 2036) and the second passivation layer (2130, 2136) are in direct electrical communication with each other allowing a current to pass from the first conductor (2020) to the second conductor (2120).

2. The shaft assembly (200) of claim 1, wherein the first reactive material and the second reactive material are the same material.

3. The shaft assembly (200) of claim 1 or 2, wherein the first reactive material is selected from the group consisting of titanium, tantalum, and niobium, and wherein the first passivation layer (2030, 2036) is a respective oxide film of at least one of titanium oxide, tantalum pentoxide, and niobium oxide.

4. The shaft assembly (200) of any one of the preceding claims, wherein the first passivation layer (2030, 2036) and the second passivation layer (2130, 2136) are each less than 200nm in thickness.

5. The shaft assembly (200) of any one of the preceding claims, wherein the first conductor (2020) is a first conductive ring around a circumference of the first slip ring (2010) and the second conductor (2120) is a second conductive ring around a circumference of the second slip ring (2110).

6. The shaft assembly (200) of any one of claims 1 to 5, wherein the first conductor (2020) is a first surface of a distal end (2034) of the first slip ring (2010), and the second conductor (2120) is a second surface of a proximal end (2134) of the second slip ring (2110).

7. The shaft assembly (200) of any one of the preceding claims, wherein the first reactive material and the second reactive material have a surface polish to increase passivation.

8. The shaft assembly (200) of any one of the preceding claims, wherein the shaft assembly (200) is disposed within a multi-use surgical instrument (10), and wherein the first conductor (2020) and the second conductor (2120) are exposed to an external environment allowing the first passivation layer (2030, 2036) and the second passivation layer (2130, 2136) to form and/or increase in thickness.

9. The shaft assembly (200) of claim 8, wherein the external environment comprises saline, and the first passivation layer (2030, 2036) and the second passivation layer (2130, 2136) are configured to form and/or increase in thickness when subjected to the saline.

10. The shaft assembly (200) of any one of the preceding claims further comprising:
a control circuit (2080) electrically connected to the first conductor (2020); and
an end effector (300) electrically connected to the second conductor (2120), wherein the control circuit (2080) is configured to communicate signals and/or power to and from the end effector (300) via the current passing between the first conductor (2020) and the second conductor (2120).

11. The shaft assembly (200) of claim 10, wherein the signals and/or power remain substantially constant as a thickness of the first passivation layer (2030, 2036) and the second passivation layer (2130, 2136) increases.

12. The shaft assembly (200) of any one of the preceding claims, wherein the current is from approximately 0.25A to approximately 0.42A.

13. The shaft assembly (200) of any one of the preceding claims, wherein the first passivation layer (2030, 2036) and the second passivation layer (2130, 2136) are pre-formed.

14. The shaft assembly (200) of any preceding claim, wherein the end effector (300) comprises a channel circuit (1670) and exposed contacts (1676) disposed thereon, wherein the exposed contacts (1676) comprise a third reactive material, third reactive material comprising a third passivation layer (2330) of oxide formed thereon.

15. The shaft assembly (200) of claim 14, wherein the first reactive material and the third reactive material are the same material.
